# EUROPEAN PATENT APPLICATION

(11) **EP 2 002 777 A2**
(43) Date of publication of application: **17.12.2008**
(21) Application number: 07739528.3
(22) Date of filing: 23.03.2007
(51) Int. Cl.: A61B 1/00, A61B 8/12

(54) **MEDICAL SYSTEM**

(30) Priority: 06.04.2006 JP 2006105637
(71) Applicant: Olympus Medical Systems Corp., Tokyo 151-0072 (JP)
(72) Inventor: HIBI, Yasushi, Shibuya-ku, Tokyo 151-0072 (JP)
(74) Representative: von Hellfeld, Axel
(86) International application number: PCT/JP2007/056087
(87) International publication number: WO 2007/114085

(57) **Abstract**

A medical system of the invention includes: a first medical apparatus for acquiring first biological information; a second medical apparatus for acquiring second biological information different from the first biological information; first instruction inputting means for providing an operational instruction to the first medical apparatus; and second instruction inputting means for providing an operational instruction to the second medical apparatus. The first instruction inputting means has common instruction inputting means for providing a common operational instruction of the operational instruction provided to the first medical apparatus and the operational instruction provided to the second medical apparatus.

## Description

### Technical Field

The present invention relates to a medical system, and more particularly to a medical system including a plurality of medical apparatuses each of which are used for acquiring different biological information.

### Background Art

Conventionally, in medical field, there have been widely used apparatuses such as an electronic endoscope image pickup apparatus (hereinafter referred to as an electronic endoscope apparatus) for performing image processing on an image of a living tissue in a living body as a subject which is picked up by an electronic endoscope, and an ultrasound diagnostic apparatus for performing image processing on a tomographic image of a living tissue in a living body as a subject which is acquired by an ultrasound endoscope. The above-described apparatuses are used individually in some cases, and used as a medical system in which a plurality of apparatuses are operated in conjunction with one another in other cases.

An ultrasound diagnostic apparatus disclosed, for example, in Japanese Patent Application Laid-Open Publication No. 07-184889, which is configured as the above-described medical system, includes an operation table capable of providing operational instructions to both of the medical apparatuses, that is, the ultrasound diagnostic apparatus and the electronic endoscope apparatus.

The operation table included in the ultrasound diagnostic apparatus disclosed in the Japanese Patent Application Laid-Open Publication No. 07-184889 has a configuration in which switches and the like used for providing operational instructions to the medical apparatuses as operation targets are mixedly provided. Therefore, in the ultrasound diagnostic apparatus disclosed in the Japanese Patent Application Laid-Open Publication No. 07-184889, in a case of performing a treatment in which one medical apparatus of the electronic endoscope apparatus and the ultrasound diagnostic apparatus is more frequently used and the other medical apparatus is less frequently used, for example, there is a problem that operability is decreased at the time of providing an operational instruction to the frequently-used medical apparatus.

The present invention has been achieved in view of the above-described points, and an object of the present invention is to provide a medical system capable of improving operability compared with a conventional system when an operator and the like provides operational instructions to a plurality of medical apparatuses.

### Disclosure of Invention

### Means for Solving the Problem

A medical system of a first aspect of the present invention comprises: a first medical apparatus for acquiring first biological information; a second medical apparatus for acquiring second biological information different from the first biological information; first instruction inputting means for providing an operational instruction to the first medical apparatus; and second instruction inputting means for providing an operational instruction to the second medical apparatus, wherein the first instruction inputting means includes common instruction inputting means for providing a common operational instruction of the operational instruction provided to the first medical apparatus and the operational instruction provided to the second medical apparatus.

A medical system of a second aspect of the present invention is the medical system of the first aspect, wherein the common instruction inputting means includes character inputting means.

A medical system of a third aspect of the present invention is the medical system of the first aspect, wherein the first medical apparatus is an electronic endoscope apparatus for acquiring an optical image of a subject as the first biological information.

A medical system of a fourth aspect of the present invention is the medical system of the second aspect, wherein the first medical apparatus is an electronic endoscope apparatus for acquiring an optical image of a subject as the first biological information.

A medical system of a fifth aspect of the present invention is the medical system of the second aspect, wherein the second medical apparatus is an ultrasound diagnostic apparatus for acquiring a tomographic image of a subject as the second biological information.

A medical system of a sixth aspect of the present invention is the medical system of the second aspect, wherein the second medical apparatus is an ultrasound diagnostic apparatus for acquiring a tomographic image of a subject as the second biological information.

A medical system of a seventh aspect of the present invention is the medical system of the third aspect, wherein the second medical apparatus is an ultrasound diagnostic apparatus for acquiring a tomographic image of the subject as the second biological information.

A medical system of an eighth aspect of the present invention is the medical system of the fourth aspect, wherein the second medical apparatus is an ultrasound diagnostic apparatus for acquiring a tomographic image of the subject as the second biological information.

A medical system of a ninth aspect of the present invention is the medical system of the fifth aspect, wherein the second instruction inputting means includes at least a trackball capable of providing an instruction to rotate the tomographic image to be displayed on a monitor.

A medical system of a tenth aspect of the present invention is the medical system of the sixth aspect, wherein the second instruction inputting means includes at least a trackball capable of providing an instruction to rotate the tomographic image to be displayed on a monitor.

A medical system of an eleventh aspect of the present invention is the medical system of the seventh aspect, wherein the second instruction inputting means includes at least a trackball capable of providing an instruction to rotate the tomographic image to be displayed on a monitor.

A medical system of a twelfth aspect of the present invention is the medical system of the eighth aspect, wherein the second instruction inputting means includes at least a trackball capable of providing an instruction to rotate the tomographic image to be displayed on a monitor.

### Brief Description of the Drawings

FIG. 1 is a view showing one example of a configuration of a main part of a medical system according to a present embodiment.
FIG. 2 is a view showing one example of an appearance of a keyboard included in the medical system in FIG. 1.
FIG. 3 is a block diagram showing one example of an internal configuration of the keyboard included in the medical system in FIG. 1.
FIG. 4 is a side view showing an appearance of the keyboard included in the medical system in FIG. 1 in a case where the keyboard is provided with an LED for illumination.
FIG. 5 is a view showing one example of a case where a layout of the keyboard included in the medical system in FIG. 1 is displayed on a monitor.
FIG. 6 is a view showing one example of an appearance of a keypad included in the medical system in FIG. 1.
FIG. 7 is a block diagram showing one example of an internal configuration of the keypad included in the medical system in FIG. 1.

### Best Mode for Carrying Out the Invention

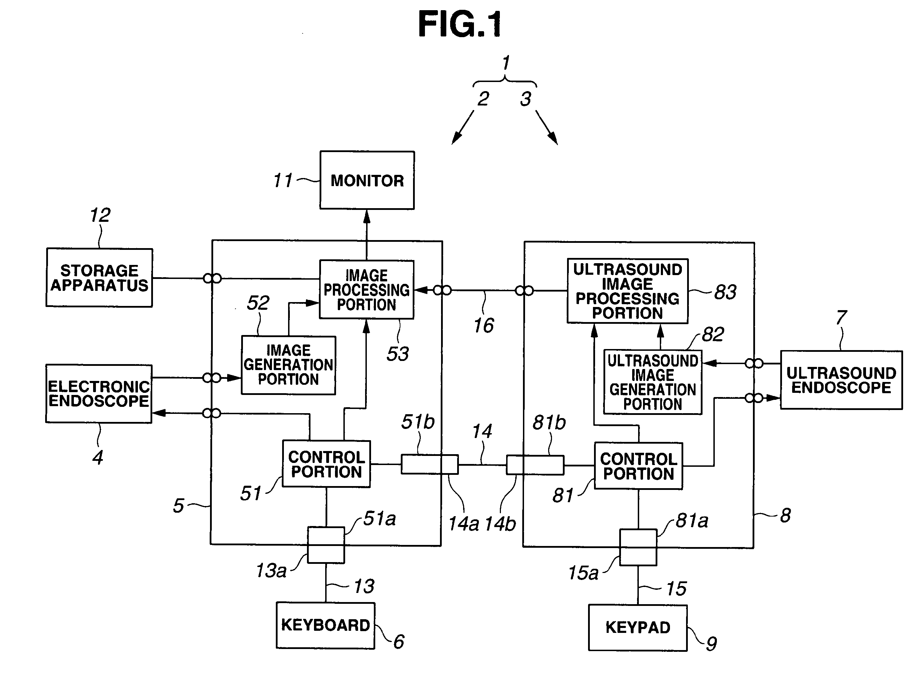

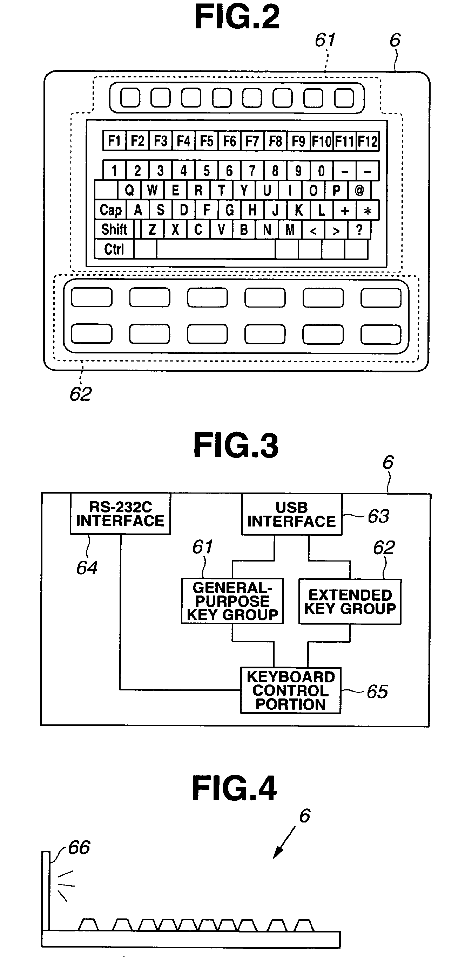

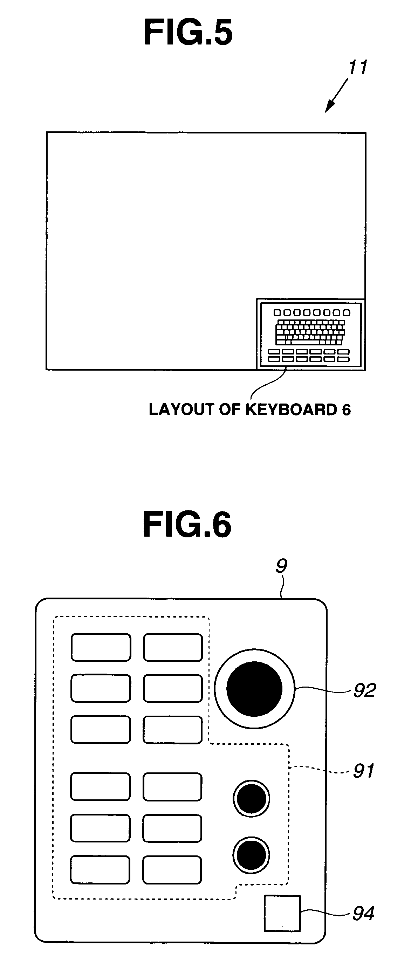

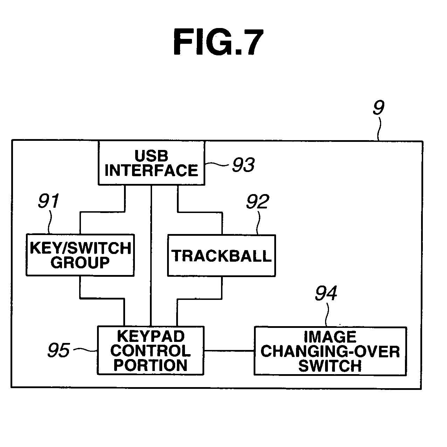

FIGS. 1 to 7 relate to an embodiment of the present invention. FIG. 1 is a view showing one example of a configuration of a main part of a medical system according to the present embodiment. FIG. 2 is a view showing one example of an appearance of a keyboard included in the medical system in FIG. 1. FIG. 3 is a block diagram showing one example of an internal configuration of the keyboard included in the medical system in FIG. 1. FIG. 4 is a side view showing an appearance of the keyboard included in the medical system in FIG. 1 in a case where the keyboard is provided with an LED for illumination. FIG. 5 is a view showing one example of a case where a layout of the keyboard included in the medical system in FIG. 1 is displayed on a monitor. FIG. 6 is a view showing one example of an appearance of a keypad included in the medical system in FIG. 1. FIG. 7 is a block diagram showing one example of an internal configuration of the keypad included in the medical system in FIG. 1.

As shown in FIG. 1, a medical system 1 has a main part configured of an electronic endoscope apparatus 2 to be connected to external apparatuses such as a monitor 11 as a display apparatus and a storage apparatus 12 configured of a portable memory and the like, and an ultrasound diagnostic apparatus 3, for example.

As shown in FIG. 1, the electronic endoscope apparatus 2 has a main part configured of: an electronic endoscope 4 provided with an image pickup device and the like, not shown, for picking up an image of a subject and outputting the picked-up image as an image pickup signal; an electronic endoscope control apparatus 5 for generating a video signal based on the image pickup signal outputted from the electronic endoscope 4 and outputting the video signal; and a keyboard 6 for providing an operational instruction and the like to the electronic endoscope control apparatus 5.

In other words, the electronic endoscope apparatus 2 is configured by including the electronic endoscope 4 and the electronic endoscope control apparatus 5 which have a configuration as a medical apparatus for acquiring an image (optical image) of the subject as biological information, and the keyboard 6 as instruction inputting means for providing operational instructions to the electronic endoscope 4 and the electronic endoscope control apparatus 5.

In addition, as shown in FIG. 1, the ultrasound diagnostic apparatus 3 has a main part configured of: an ultrasound endoscope 7 provided with an ultrasound transducer and the like, not shown, which transmits ultrasound waves to the subject and receives reflected waves caused by the ultrasound waves reflected from the subject and outputs the received reflected waves as an echo signal; an ultrasound endoscope control apparatus 8 for generating a video signal based on the echo signal outputted from the ultrasound endoscope 7 and outputting the video signal; and a keypad 9 for providing an operational instruction and the like to the ultrasound endoscope control apparatus 8.

In other words, the ultrasound endoscope apparatus 3 is configured by including the ultrasound endoscope 7 and the ultrasound endoscope control apparatus 8, which have a configuration as a medical apparatus for acquiring a tomographic image of the subject as biological information, and the keypad 9 as instruction inputting means for providing operational instructions to the ultrasound endoscope 7 and the ultrasound endoscope control apparatus 8.

As shown in FIG. 1, the electronic endoscope control apparatus 5 has a main part configured of a control portion 51 connected to communications interfaces 51a and 51 b included in the electronic endoscope control apparatus 5, an image generation portion 52, and an image processing portion 53.

The control portion 51 configured of a CPU and the like outputs control signals for controlling the electronic endoscope 4 and an image processing portion 53, based on an operational instruction signal outputted from the keyboard 6 through a cable 13 connected to the communications interface 51a and an image changing-over control signal outputted from the ultrasound endoscope control apparatus 8 through a cable 14 connected to the communications interface 51b. In addition, based on the image changing-over control signal outputted from the ultrasound endoscope control apparatus 8, the control portion 51 outputs the operational instruction signal outputted from the keyboard 6, through the cable 14 connected to the communications interface 51b.

The communications interface 51a includes a connector-connecting terminal pursuant to a communications standard such as USB (Universal Serial Bus), RS-232C, or the like, and is configured to be connectable with a connector 13a provided at an end portion of the cable 13 which has a configuration suitable for the communications standard. The electronic endoscope control apparatus 5 is connected to the keyboard 6 through the communications interface 51a and the cable 13 which have the above-described configurations.

The communications interface 51 b includes a connector-connecting terminal pursuant to the communications standard such as the USB, RS-232C, or the like, and is configured to be connectable with a connector 14a provided at an end portion of the cable 14 having a configuration suitable for the communications standard.

The image generation portion 52 generates an image of the subject based on the image pickup signal outputted from the electronic endoscope 4 and outputs the image to the image processing portion 53 as an image signal.

The image processing portion 53 performs an image processing on the image signal outputted from the image generation portion 52 based on the control signal outputted from the control portion 51, and when an instruction is then provided through the keypad 9 to display the image of the subject based on the image signal on the monitor 11, the image processing portion 53 converts the image signal into a video signal and outputs the video signal to the monitor 11. Furthermore, when an instruction is provided through a key pad 9 to display on the monitor 11 a tomographic image of the subject based on the image signal outputted from the ultrasound endoscope control apparatus 8, the image processing portion 53 converts the image signal into a video signal and outputs the video signal to the monitor 11, based on the control signal outputted from the control portion 51.

When an operational instruction is provided through the keyboard 6 to store the image of the subject based on the image pickup signal outputted from the electronic endoscope 4, the image processing portion 53 outputs an image signal outputted from the image generation portion 52 to the storage apparatus 12, based on the control signal outputted from the control portion 51. Furthermore, when an operational instruction is provided through the keyboard 6 to store the tomographic image of the subject based on the echo signal outputted from the ultrasound endoscope 7, the image processing portion 53 outputs the image signal outputted from the ultrasound endoscope control apparatus 8 to the storage apparatus 12, based on the control signal outputted from the control portion 51.

The keyboard 6 as the instruction inputting means has the appearance shown in FIG. 2 and the internal configuration shown in FIG. 3, for example. Specifically, as shown in FIG. 3, the keyboard 6 includes a general-purpose key group 61, an extended key group 62, a USB interface 63, an RS-232c interface 64, and a keyboard control portion 65.

The general-purpose key group 61 as common instruction inputting means is configured pursuant to, for example, the USB-HID (Human Interface Device) standard as a predetermined standard. In addition, the general-purpose key group 61 includes a plurality of keys commonly used both in the electronic endoscope apparatus 2 and the ultrasound diagnostic apparatus 3. Specifically, the general-purpose key group 61 includes, for example, alphanumeric keys for inputting characters, and a release key for providing an instruction to store one of the images to be displayed on the monitor 11, that is, either one of the image of the subject based on the image pickup signal outputted from the electronic endoscope 4 and the tomographic image of the subject based on the echo signal outputted from the ultrasound endoscope 7. The general-purpose key group 61 outputs operational instruction signals based on the character inputted and instructions provided through the plurality of keys to the USB interface 63 and the keyboard control portion 65.

The extended key group 62 is configured pursuant to, for example, the USB-HID standard as a predetermined standard. Furthermore, the extended key group 62 includes one or a plurality of keys for providing various kinds of operational instructions to the electronic endoscope 4. The extended key group 62 outputs operational instruction signals based on the instruction provided through the plurality of keys to the USB interface 63 and the keyboard control portion 65.

The USB interface 63 includes a connector-connecting terminal, a hub, and the like, which are pursuant to the USB communications standard. Furthermore, when the electronic endoscope control apparatus 5 and the keyboard 6 are USB-connected by the cable 13, the USB interface 63 outputs to the electronic endoscope control apparatus 5 the operational instruction signals outputted from the general-purpose key group 61 and the extended key group 62.

The RS-232C interface 64 includes a connector-connecting terminal and the like, which are pursuant to the RS-232C communications standard. Furthermore, when the electronic endoscope control apparatus 5 and the keyboard 6 are RS-232C-connected by the cable 13, the RS-232C interface 64 converts the operational instruction signals outputted from the general-purpose key group 61 and the extended key group 62 through the keyboard control portion 65 into signals pursuant to the RS-232C and outputs the operational instruction signals subjected to the conversion to the electronic endoscope control apparatus 5.

As described above, the keyboard 6 is provided with the two communications interfaces, the USB interface 63 and the RS-232C interface 64. Therefore, the keyboard 6 can be connected to the electronic endoscope control apparatus 5 in more various ways compared with a keyboard including only a single communications interface.

When the electronic endoscope control apparatus 5 and the keyboard 6 are RS-232C-connected by the cable 13, the keyboard control portion 65 configured of a CPU and the like performs a control to output to the RS-232C interface 64 the operational instruction signals outputted from the general-purpose key group 61 and the extended key group 62.

Note that, in order to improve visibility when the keyboard 6 is used in a dark place in particular, the keyboard 6 may have at one end thereof an LED 66 for illumination, turning on and off of which can be switched by one key belonging to the general-purpose key group 61, as shown in FIG. 4, for example.

In addition, in order to improve the visibility when used in a dark place in particular, the keyboard 6 may have a configuration in which a layout thereof is displayed at one portion on the screen of the monitor 11, as shown in FIG. 5, for example.

As shown in FIG. 1, the ultrasound endoscope control apparatus 8 has a main part configured of: a control portion 81 connected to communications interfaces 81a, 81 b included in the ultrasound endoscope control apparatus 8; an ultrasound image generation portion 82; and an ultrasound image processing portion 83.

The control portion 81 configured of a CPU and the like outputs control signals for controlling the ultrasound endoscope 7 and the ultrasound image processing portion 83, based on the operational instruction signal and the image changing-over control signal outputted from the keypad 9 through the cable 15 connected to the communications interface 81a. In addition, the control portion 81 outputs the image changing-over control signal outputted from the keypad 9 to the electronic endoscope control apparatus 5, through the cable 14 connected to the communications interface 81b.

The communications interface 81a includes a connector-connecting terminal pursuant to a communications standard such as the USB, for example, and is configured to be connectable with a connector 15a provided at an end portion of the cable 15 which has a configuration suitable for the communications standard. The ultrasound endoscope control apparatus 8 is connected to the keypad 9 through the communications interface 81 a and the cable 15 which have the above-described configuration.

The communications interface 81b includes a connector-connecting terminal pursuant to a communications standard such as the USB or RS-232C, for example, and is configured to be connectable with a connector 14b provided at the end portion of the cable 14 which has a configuration suitable for the communications standard.

The ultrasound image generation portion 82 generates a tomographic image of the subject based on the echo signal outputted from the ultrasound endoscope 7, and outputs the image to the ultrasound image processing portion 83 as an image signal.

The ultrasound image processing portion 83 performs an image processing on the image signal outputted from the ultrasound image generation portion 82, based on the control signal outputted from the control portion 81. After that, the ultrasound image processing portion 83 outputs the image signal to the image processing portion 53 through a cable 16.

The keypad 9 as instruction inputting means has an appearance as shown in FIG. 6 and an inner configuration as shown in FIG. 7, for example. Specifically, as shown in FIG. 7, the keypad 9 includes a key/switch group 91, a trackball 92, a USB interface 93, an image changing-over switch 94, and a keypad control portion 95.

The key/switch group 91 is configured pursuant to, for example, the USB-HID standard as a predetermined standard. In addition, the key/switch group 91 includes, for example, one or a plurality of keys for providing various operational instructions to the ultrasound endoscope 7. The key/switch group 91 outputs to the USB interface 93 the operational instruction signals based on the instructions provided by the plurality of keys.

The trackball 92 is configured pursuant to the USB-HID standard as a predetermined standard, for example. The trackball 92, for example, outputs to the USB interface 93 an operational instruction signal based on an instruction to rotate the tomographic image of the subject to be displayed on the monitor 11, which is based on the echo signal outputted from the ultrasound endoscope 7.

The USB interface 93 includes a connector-connecting terminal, hub, and the like pursuant to the USB communications standard. In addition, when the ultrasound endoscope control apparatus 8 and the keypad 9 are connected to each other by the cable 15, the USB interface 93 outputs to the ultrasound endoscope control apparatus 8 the operational instruction signals outputted from the key/switch group 91 and the trackball 92.

The image changing-over switch 94 outputs to the keypad control portion 95 an image changing-over instruction signal to change over an image to be displayed on the monitor 11 to either one of the image of the subject based on the image pickup signal outputted from the electronic endoscope 4 or the tomographic image of the subject based on the echo signal outputted from the ultrasound endoscope 7. Note that the image changing-over switch 94 is not limited to one provided to the keypad 9 if only having the above-described configuration to output the image changing-over instruction signal, and may be, for example, provided to the keyboard 6 and the like.

Based on the image changing-over instruction signal outputted from the image changing-over switch 94, the keypad control portion 95, for example, allows the image of the subject based on the image pickup signal outputted from the electronic endoscope 4 to be displayed on the monitor 11, and outputs to the control portion 51 of the electronic endoscope control apparatus 5 an image changing-over control signal including an instruction to superimpose the content of the character input performed through the alphanumeric keys included in the general-purpose key group 61 on the image of the subject, through the cable 15, the ultrasound endoscope control apparatus 8, and the cable 14.

In addition, based on the image changing-over instruction signal outputted from the image changing-over switch 94, the keypad control portion 95, for example, allows the tomographic image of the subject based on the echo signal outputted from the ultrasound endoscope 7 to be displayed on the monitor 11, and outputs to the control portion 81 of the ultrasound endoscope control apparatus 8 an image changing-over control signal including an instruction to superimpose the content of the character input performed through the alphanumeric keys included in the general-purpose key group 61 on the tomographic image of the subject, through the USB interface 93 and the cable 15. Furthermore, the keypad control portion 95 outputs the image changing-over control signal also to the control portion 51 of the electronic endoscope control apparatus 5, through the USB interface 93, the cable 15, the ultrasound endoscope control apparatus 8, and the cable 14.

Next, a working of the medical system 1 according to the present embodiment is described.

First, an operator or the like connects the cables 13, 14, 15, and 16 to respective portions in the medical system 1, and thereafter turns on powers to start up the portions. Note that, hereinafter, for simplification of description, it is assumed that the electronic endoscope control apparatus 5 and the keyboard 6 are USB-connected by the cable 13.

After that, in a state where the image of the subject based on the image pickup signal outputted from the electronic endoscope 4 is displayed on the monitor 11, when an operator or the like inputs characters by operating the alphanumeric keys included in the general-purpose key group 61 on the keyboard 6, for example, the general-purpose key group 61 outputs to the electronic endoscope control apparatus 5 an operational instruction signal based on the character input, through the USB interface 63 and the cable 13.

Based on the operational instruction signal outputted from the keyboard 6, the control portion 51 of the electronic endoscope control apparatus 5 outputs a control signal to perform a control of superimposing the content of the character input performed through the keyboard 6 on the image of the subject based on the image pickup signal outputted from the electronic endoscope 4, to the image processing portion 53.

Based on the control signal outputted from the control portion 51, the image processing portion 53 performs an image processing of superimposing the content of the character input performed through the keyboard 6 on the image signal outputted from the image generation portion 52, and thereafter converts the image signal subjected to the image processing into a video signal to output the video signal to the monitor 11.

By the above-described working, the content of the character input performed through the keyboard 6 is displayed on the monitor 11 in a superimposed manner on the image of the subject based on the image pickup signal outputted from the electronic endoscope 4.

After that, in a state where the image of the subject based on the image pickup signal outputted from the electronic endoscope 4 is displayed on the monitor 11, when the operator or the like provides an instruction to display the tomographic image of the subject based on the echo signal outputted from the ultrasound endoscope 7 on the monitor 11 by operating the image changing-over switch 94 on the keypad 9, the image changing-over switch 94 outputs an image changing-over instruction signal based on the instruction to the keypad control portion 95.

The keypad control portion 95 allows the tomographic image of the subject based on the echo signal outputted from the ultrasound endoscope 7 to be displayed on the monitor 11 based on the image changing-over instruction signal outputted from the image changing-over switch 94, and outputs an image changing-over control signal including the instruction to superimpose the content of the character input performed through the alphanumeric keys in the general-purpose key group 61 on the tomographic image of the subject, to the control portion 81 of the ultrasound endoscope control apparatus 8 and the control portion 51 of the electronic endoscope control apparatus 5, through the cable 15 and the like.

Based on the image changing-over control signal outputted from the ultrasound endoscope control apparatus 8, the control portion 51 outputs an operational instruction signal which is a signal including the content of the character input performed through the keyboard 6, to the control portion 81 of the ultrasound endoscope apparatus 8, through the cable 14. In addition, based on the image changing-over control signal outputted from the ultrasound endoscope control apparatus 8, the control portion 51 outputs to the image processing portion 53 a control signal to perform a control to allow the tomographic image of the subject based on the echo signal outputted from the ultrasound endoscope 7 to be outputted on the monitor 11.

Based on the operational instruction signal outputted from the electronic endoscope control apparatus 5 through the cable 14 and the image changing-over control signal outputted from the keypad 9, the control portion 81 outputs to the ultrasound image processing portion 83 a control signal for performing a control to superimpose the content of the character input performed through the keyboard 6 on the tomographic image of the subject based on the echo signal outputted from the ultrasound endoscope 7.

Based on the control signal outputted from the control portion 81, the ultrasound image processing portion 83 performs an image processing to superimpose the content of the character input performed through the keyboard 6 on the image signal outputted from the ultrasound image generation portion 82, and thereafter outputs the image signal subjected to the image processing to the image processing portion 53 of the electronic endoscope control apparatus 5 through the cable 16.

Based on the control signal outputted from the control portion 51, the image processing portion 53 converts the image signal outputted from the ultrasound endoscope control apparatus 8 into a video signal and outputs the video signal to the monitor 11.

By the above-described working, the content of the character input performed through the keyboard 6 is displayed on the monitor 11 in a superimposed manner on the tomographic image of the subject based on the echo signal outputted from the ultrasound endoscope 7.

Note that the transmission and reception of the above-described operational instruction signal and the image changing-over control signal are not necessarily performed between the electronic endoscope control apparatus 5 and the ultrasound endoscope control apparatus 8 through the cable 14. Specifically, the above-described operational instruction signal and the image changing-over control signal may be transmitted and received between the keyboard 6 and the keypad 9 through a cable, not shown, such as a USB cable which is configured to be connectable to the USB interfaces 63 and 93, for example, and thereafter may be outputted to the electronic endoscope control apparatus 5 or to the ultrasound endoscope control apparatus 8.

By the above-described working, the content of the character input performed through the keyboard 6 is displayed on the monitor 11 in a superimposed manner on the tomographic image of the subject based on the echo signal outputted from the ultrasound endoscope 7.

As described above, in the medical system 1 according to the present embodiment, among the operational instructions provided to each of the portions in the electronic endoscope apparatus 2 and the ultrasound diagnostic apparatus 3, the operational instruction which is preferably provided independently in each of the apparatuses, for example, the operational instruction with respect to the electronic endoscope 4 and the operational instruction with respect to the ultrasound endoscope 7 can be provided only by the keyboard 6 and by the keypad 9, respectively. As a result, even when performing a treatment in which one medical apparatus is more frequently used and the other medical apparatus is less frequently used, the medical system 1 according to the present embodiment is capable of preventing decrease in operability in providing an operational instruction to the frequently-used medical apparatus.

In addition, in the medical system 1 according to the present embodiment, operational instructions which can be commonly provided in the electronic endoscope apparatus 2 and ultrasound diagnostic apparatus 3, for example, the operational instructions of character input and image storage can be provided through the keyboard 6. As a result, the medical system 1 according to the present embodiment can improve operability for the operator or the like to provide operational instructions to a plurality of medical apparatuses, compared with a conventional system.

Note that, it is needless to say that the present invention is not limited to the above described embodiment, and various modifications and applications are possible in an implementation stage within the scope of the present invention.

The present application is based on the priority of Japanese Patent Application No. 2006-105637 filed on April 6, 2006. The disclosure is referred to the description, claims, and drawings of the present invention.

## Claims

1. A medical system comprising:
a first medical apparatus for acquiring first biological information;
a second medical apparatus for acquiring second biological information different from the first biological information;
first instruction inputting means for providing an operational instruction to the first medical apparatus; and
second instruction inputting means for providing an operational instruction to the second medical apparatus,
wherein the first instruction inputting means includes common instruction inputting means for providing a common operational instruction of the operational instruction provided to the first medical apparatus and the operational instruction provided to the second medical apparatus.

2. The medical system according to claim 1, wherein the common instruction inputting means includes character inputting means.

3. The medical system according to claim 1, wherein the first medical apparatus is an electronic endoscope apparatus for acquiring an optical image of a subject as the first biological information.

4. The medical system according to claim 2, wherein the first medical apparatus is an electronic endoscope apparatus for acquiring an optical image of a subject as the first biological information.

5. The medical system according to claim 1, wherein the second medical apparatus is an ultrasound diagnostic apparatus for acquiring a tomographic image of a subject as the second biological information.

6. The medical system according to claim 2, wherein the second medical apparatus is an ultrasound diagnostic apparatus for acquiring a tomographic image of a subject as the second biological information.

7. The medical system according to claim 3, wherein the second medical apparatus is an ultrasound diagnostic apparatus for acquiring a tomographic image of the subject as the second biological information.

8. The medical system according to claim 4, wherein the second medical apparatus is an ultrasound diagnostic apparatus for acquiring a tomographic image of the subject as the second biological information.

9. The medical system according to claim 5, wherein the second instruction inputting means includes at least a trackball capable of providing an instruction to rotate the tomographic image to be displayed on a monitor.

10. The medical system according to claim 6, wherein the second instruction inputting means includes at least a trackball capable of providing an instruction to rotate the tomographic image to be displayed on a monitor.

11. The medical system according to claim 7, wherein the second instruction inputting means includes at least a trackball capable of providing an instruction to rotate the tomographic image to be displayed on a monitor.

12. The medical system according to claim 8, wherein the second instruction inputting means includes at least a trackball capable of providing an instruction to rotate the tomographic image to be displayed on a monitor.
